# FASCICULE DE BREVET EUROPEEN

(11) **EP 0 562 967 B1**
(45) Date de publication et mention de la délivrance du brevet: **11.06.1997**
(21) Numéro de dépôt: 93400775.8
(22) Date de dépôt: 25.03.1993
(51) Int. Cl.: A61F 5/01, A61F 13/06

(54) **Chevillère, notamment pour le sport**
Knöchelsocke, insbesondere für Sport
Anklet, in particular for sports

(30) Priorité: 26.03.1992 FR 9203641
(43) Date de publication de la demande: 29.09.1993
(73) Titulaire: ETABLISSEMENTS THUASNE & CIE, F-42031 St. Etienne Cedex (FR); THUASNE, 92300 Levallois-Perret (FR)
(72) Inventeur: Ducottet, Elisabeth, F-92200 Neuilly Sur Seine (FR); Picolet, Jean-Pierre, F-42270 Saint Priest en Jarez (FR); Courtet, François, F-43330 Saint Galmier (FR)
(74) Mandataire: Coutel, Jean-Claude

(56) Documents cités:
- BE-A- 885 515
- FR-A- 2 607 383
- GB-A- 2 068 710
- US-A- 2 592 739

## Description

La présente invention est relative à une chevillère de protection, et elle s'applique en particulier à une telle chevillère qui est utilisée dans la pratique des activités sportives pour protéger la zone de la cheville, en particulier les malléoles et le tendon d'Achille, contre les coups.

De manière plus particulière encore, l'invention s'applique à une telle chevillère qui est utilisée en association avec un protège-tibia, notamment pour la pratique du football.

Les chevillères actuellement connues sont fabriquées par tricotage d'un tube qui, d'une part, est d'abord découpé sur son bord supérieur et sur son bord inférieur pour présenter sur chaque bord deux excroissances et, d'autre part, est ensuite replié sur lui-même ou doublé pour constituer un tube d'une longueur égale à la moitié du tube initial, les excroissances des deux bords venant en superposition respectivement et étant destinées à protéger les malléoles. Le tube ainsi doublé est ensuite cousu sur son bord inférieur profilé, après insertion entre les deux épaisseurs de tricot d'une mousse de protection destinée à protéger le tendon d'Achille et les malléoles.

Ces chevillères connues présentent essentiellement deux inconvénients d'ordre économique. D'une part, il est nécessaire de tricoter initialement un tube d'une longueur double de celle du tube final, ce qui entraîne un prix de revient de matière double par rapport à celui d'une chevillère présentant une couche unique ; d'autre part, les opérations de couture du tube initial et de couture du tube final ne peuvent pas se faire à plat, ce qui rend ces opérations particulièrement délicates et coûteuses.

Par le document FR-A-2.607.383, on connaît par ailleurs un appareil de contention du tendon d'Achille qui comporte, d'une part, une pièce de tissu élastique fabriquée à plat et refermée sur elle-même pour former un tube et, d'autre part, une pièce souple assujettie à la pièce de tissu et agencée pour recouvrir au moins le tendon d'Achille. Dans cet appareil, la pièce souple, constituée par une partie du tissu élastique, recouvre simplement le tendon d'Achille sans le protéger, et on prévoit deux pelotes, de part et d'autre de ce dernier, qui sont logées dans les creux situés derrière les malléoles et dont le rôle est d'avoir une fonction de contention du tendon, notamment pour amortir les vibrations. Cet appareil a donc une fonction de simple contention transversale du tendon, sans le protéger par l'arrière ; au surplus, il n'a aucune fonction de protection des malléoles, notamment contre les chocs.

L'invention a pour but de remédier à ces inconvénients en fournissant une chevillère qui n'implique pas un excédent de matière, qui puisse être fabriquée à plat et qui protège efficacement le tendon d'Achille et les malléoles.

A cet effet, la chevillère selon l'invention comportant, d'une part, une pièce de tissu élastique fabriquée à plat et refermée sur elle-même pour former un tube et, d'autre part, une pièce souple assujettie à la pièce de tissu et agencée pour recouvrir au moins le tendon d'Achille, est caractérisée en ce que la pièce de tissu est d'une forme sensiblement rectangulaire et présente, sur un grand côté, un renflement central de recouvrement de la partie supérieure du tendon d'Achille et, sur le grand côté opposé, deux renflements espacés et sensiblement symétriques de recouvrement des malléoles, et en ce que la pièce souple est une pièce de renfort et de protection recouvrant également les malléoles.

Avec la chevillère ci-dessus selon l'invention, la pièce de tissu initiale peut être découpée à plat, et la pièce de renfort peut être assujettie à celle-ci par une opération, par exemple de couture, qui peut également être effectuée à plat, ce qui rend le prix de revient des opérations de fabrication particulièrement intéressant.

Avantageusement, la pièce de tissu est élastique au moins dans le sens transversal du tissu pour exercer un effet de contention et faciliter l'enfilage.

De préférence, les deux petits côtés de la pièce de tissu sont légèrement convergents vers le haut pour que, à l'assemblage de la chevillère, celle-ci présente une forme légèrement tronconique s'évasant vers le bas.

La pièce de tissu est refermée sur elle-même, soit de manière inséparable, par exemple par une couture, soit de manière séparable, par exemple par une fermeture du type auto-agrippant.

Quant à la pièce de renfort, elle est sensiblement triangulaire, les parties d'angle étant destinées à recouvrir respectivement la partie supérieure du tendon d'Achille et les deux malléoles;cette pièce de renfort est par exemple en mousse thermocomprimée, ce qui permet de lui conférer des lignes en creux pour sa souplesse. Cette pièce de renfort peut présenter un canal arrière vertical dans la zone de coopération avec le tendon d'Achille.

La pièce de renfort est assujettie sur la face extérieure de la pièce de tissu et elle est assujettie à celle-ci sur son contour, de préférence par une couture.

Enfin, la pièce de tissu est avantageusement agencée pour recevoir un protège-tibia, de manière amovible ou fixe ; dans le cas où le protège-tibia est assujetti à demeure à la pièce de tissu, la fixation peut se faire par une couture sur le bord supérieur avant de la pièce de tissu.

On comprendra bien l'invention à la lecture du complément de description qui va suivre et en référence aux dessins annexés qui font partie de la description et dans lesquels :
Fig. 1 est une vue en perspective montrant une chevillère selon un mode de réalisation préféré de l'invention mise en place sur la cheville du porteur ;
Fig. 2 est une vue en élévation avant de la chevillère de la Fig. 1, à laquelle est associé un protège-tibia ;
Fig. 3 est une vue en élévation de la pièce de tissu de la chevillère des Figs. 1 et 2 ;
Fig. 4 est une vue en élévation de la pièce de renfort de la chevillère de la Fig. 1 ;
Fig. 5 est une vue en coupe suivant la ligne brisée V-V de la pièce de renfort de la Fig. 4 ;
Fig. 6 est une vue analogue à la Fig. 1 et relative à une variante de réalisation ;
Fig. 7 est, à plus grande échelle, une coupe transversale de la pièce de renfort de la chevillère de la Fig. 6 ; et
Fig. 8 est une coupe transversale de la chevillère de la Fig. 6.

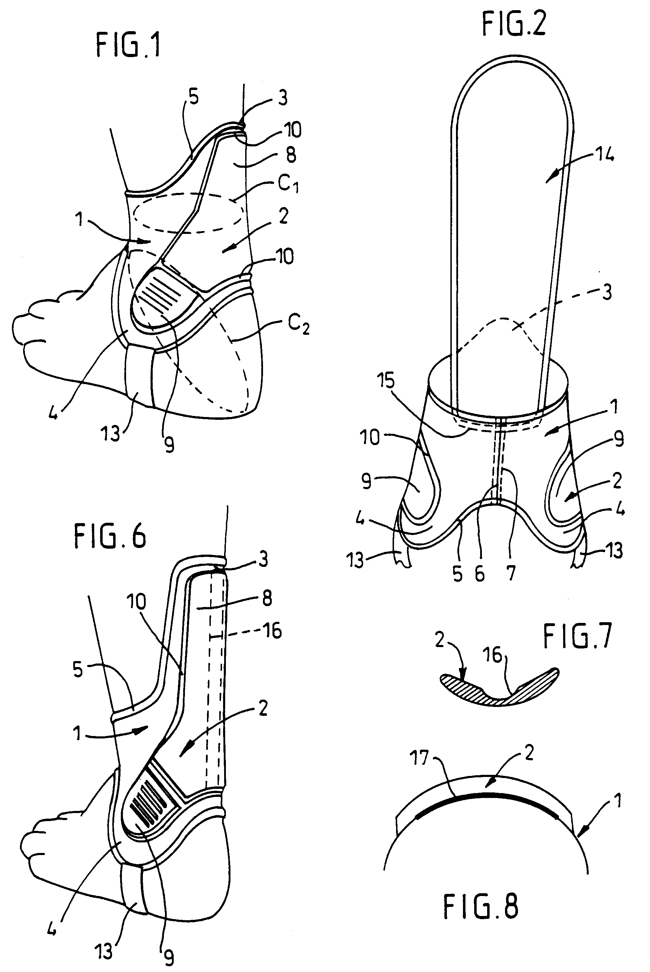

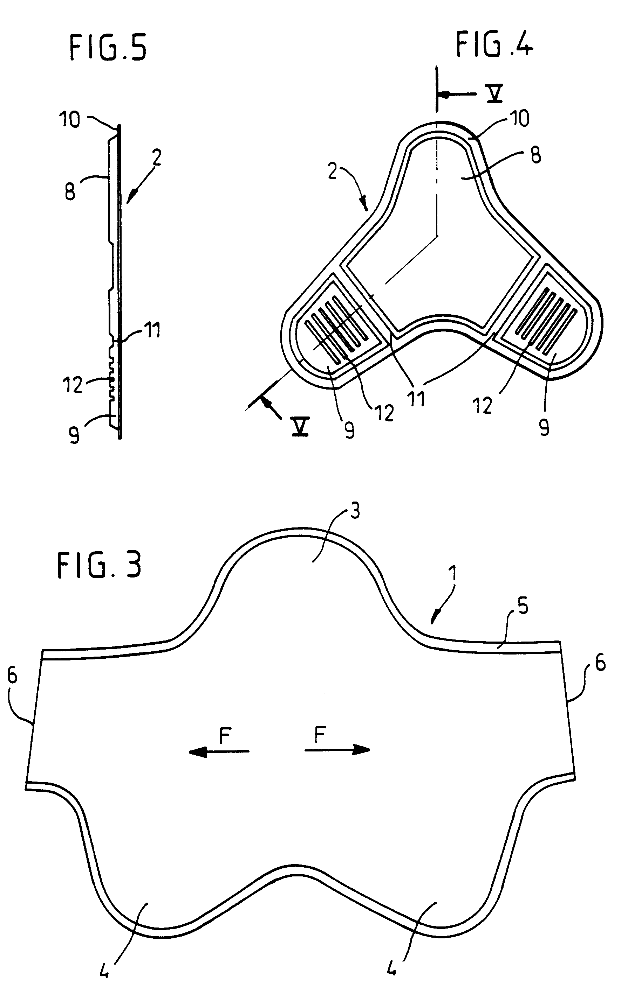

La chevillère selon l'invention, comme montré aux Figs. 1 , 3 et 4, est constituée essentiellement de deux éléments, à savoir une pièce de tissu 1 et une pièce souple de renfort 2.

La pièce de tissu 1 est constituée par un tricot fabriqué à plat qui présente une élasticité au moins dans le sens transversal, comme montré par les flèches F de la Fig. 3. Comme montré sur cette Fig. 3, la pièce 1 présente, dans l'ensemble, une forme générale sensiblement rectangulaire, un renflement ou excroissance 3 étant prévu au milieu de l'un de ses grands côtés, tandis que deux renflements ou excroissances 4 symétriques sont prévus sur l'autre grand côté. Le renflement 3 est destiné à recouvrir la partie supérieure du tendon d'Achille, tandis que les renflements 4 sont destinés à recouvrir les malléoles. La pièce de tissu 1 représentée sur la Fig. 3 est obtenue par découpe d'une pièce tricotée à plat. Le contour de la pièce 1 présente un surjet 5 évitant son effilochage.

Les deux petits côtés 6 de la pièce de tissu 1 sont convergents vers le haut pour que, quand la pièce est refermée sur elle-même, elle présente une forme tronconique s'évasant vers le bas, comme montré sur la Fig. 2.

La pièce de tissu 1 est refermée sur elle-même soit de manière inséparable, comme montré sur la Fig. 2, par exemple par des coutures 7, soit de manière séparable , par exemple en faisant appel à une fermeture du type auto-agrippant.

L'élasticité de la pièce 1 dans le sens transversal permet, comme montré sur la Fig. 1, le passage du pied, essentiellement du talon, à travers la chevillère, le périmètre de la chevillère pouvant augmenter de la valeur C₁ à la valeur C₂ correspondant au passage du talon. Par exemple, la capacité d'allongement de la pièce de tissu 1 est de 200 %.

La pièce de renfort 2, comme montré au mieux sur la Fig. 4, est d'une forme générale triangulaire, ou en étoile à trois branches, une partie d'angle 8 étant destinée à recouvrir la partie supérieure du tendon d'Achille, tandis que les deux autres parties d'angle 9 sont destinées à recouvrir et protéger les malléoles. A l'assemblage de la pièce de tissu 1 et de la pièce de renfort 2, les parties 8 et 9 de la pièce de renfort viennent se loger respectivement dans les renflements 3 et 4 de la pièce de tissu 1.

Avantageusement, la pièce de renfort 2 est en mousse thermocomprimée et elle présente une bordure périphérique plane 10 permettant sa fixation par couture sur la face extérieure de la pièce de tissu 1. La pièce de renfort 2 est en mousse thermocomprimée et elle présente avantageusement, dans les zones 9,9, des lignes en creux 11 et 12 lui conférant une souplesse supplémentaire dans la zone des malléoles.

La chevillère ainsi établie est avantageusement complétée par une bride de sous-pied 13 lui évitant de remonter.

La chevillère décrite ci-dessus peut être utilisée telle quelle ou, de préférence, en association avec un protège-tibia 14 qui coopère avec la chevillère par son bord inférieur ; cette coopération est soit amovible, le protège-tibia étant alors glissé par sa partie inférieure sous le bord avant supérieur de la chevillère, soit de manière fixe, par exemple par une couture 15, comme montré sur la Fig. 2.

On a montré sur les Figs. 6 à 8 une variante de réalisation de la chevillère, qui ne diffère de la précédente que par la conformation de la partie arrière de la pièce de tissu 1 et de la pièce de renfort 2. Dans ce cas, le renflement 3 de la pièce de tissu 1 remonte beaucoup plus haut et le renflement 8 de la pièce de renfort 2 est également situé beaucoup plus haut que précédemment, de manière à protéger encore mieux le tendon d'Achille. Dans ce cas, comme montré sur la Fig. 7, la pièce de renfort 2 peut présenter un canal vertical central 16 de coopération avec le tendon d'Achille.

Comme montré sur la Fig. 8, on peut insérer une pièce 17 en matière plastique entre la pièce de tissu 1 et la pièce souple de renfort 2.

Il ressort de la description ci-dessus que la chevillère selon l'invention peut être fabriquée et assemblée pour l'essentiel à plat, ce qui permet d'obtenir un prix de revient très intéressant.

Bien entendu, l'invention n'est pas limitée aux modes de réalisation, non plus qu'au mode d'application, qui ont été décrits ; on pourrait au contraire concevoir diverses variantes sans sortir pour autant de son cadre.

## Revendications

1. Chevillère, notamment pour le sport, comportant, d'une part, une pièce de tissu élastique (1) fabriquée à plat et refermée sur elle-même pour former un tube et, d'autre part, une pièce souple (2) assujettie à la pièce de tissu (1) et agencée pour recouvrir au moins le tendon d'Achille, caractérisée en ce que la pièce de tissu (1) est d'une forme sensiblement rectangulaire et présente, sur un grand côté, un renflement central (3) de recouvrement de la partie supérieure du tendon d'Achille et, sur le grand côté opposé, deux renflements espacés et sensiblement symétriques (4) de recouvrement des malléoles, et en ce que la pièce souple (2) est une pièce de renfort et de protection recouvrant également les malléoles.

2. Chevillère selon la revendication 1, caractérisée en ce que la pièce de tissu (1) est élastique au moins dans le sens transversal du tube.

3. Chevillère selon l'une des revendications 1 et 2, caractérisée en ce que la pièce de tissu (1) est constituée par un tricot tricoté à plat et découpé.

4. Chevillère selon l'une des revendications 1 à 3, caractérisée en ce que les deux petits côtés (6) du rectangle sont légèrement convergents vers le haut.

5. Chevillère selon l'une des revendications 1 à 4, caractérisée en ce que la pièce de tissu (1) est refermée sur elle-même de manière inséparable, par exemple par couture (7).

6. Chevillère selon l'une des revendications 1 à 4, caractérisée en ce que la pièce de tissu (1) est refermée sur elle-même de manière séparable, par exemple par une fermeture du type auto-agrippant.

7. Chevillère selon l'une des revendications 1 à 6, caractérisée en ce que la pièce de renfort (2) est sensiblement triangulaire, les parties d'angle (8,9) du triangle étant destinées à recouvrir respectivement la partie supérieure du tendon d'Achille et les deux malléoles.

8. Chevillère selon l'une des revendications 1 à 7, caractérisée en ce que la pièce de renfort (2) est en mousse thermocomprimée.

9. Chevillère selon l'une des revendications 1 à 8, caractérisée en ce que la pièce de renfort (2) présente des lignes en creux (11,12) lui conférant sa souplesse.

10. Chevillère selon l'une des revendications 1 à 9, caractérisée en ce que la pièce de renfort (2) présente un canal vertical central dans la zone de coopération avec le tendon d'Achille.

11. Chevillère selon l'une des revendications 1 à 10, caractérisée en ce que la pièce de renfort (2) est assujettie sur la face extérieure de la pièce de tissu (1), par exemple par couture.

12. Chevillère selon l'une des revendications 1 à 11, caractérisée en ce que la pièce de tissu (1) est agencée pour recevoir un protège-tibia (14), de manière amovible ou fixe, par exemple par couture (15) sur le bord supérieur avant de la pièce de tissu (1).

## Claims

1. Anklet, in particular for sports, comprising on one hand a resilient fabric part (1) fabricated flat and closed on itself to form a tube and, on another hand, a flexible part (2) secured to the fabric part (1) and arranged to cover at least the Achilles tendon, characterized in that the fabric part (1) has a substantially rectangular shape and comprises, on one long side, a central bulge (3) for covering the upper portion of the Achilles tendon and, on the opposite long side, two spaced and substantially symmetrical bulges (4) for covering the malleoluses, and in that the flexible part (2) is a reinforcement and protection part covering also the malleoluses.

2. Anklet according to claim 1, characterized in that the fabric part (1) is resilient at least in the transversal direction of the tube.

3. Anklet according to one of claims 1 and 2, characterized in that the fabric part (1) is formed by a flat-knitted and cut knitted fabric.

4. Anklet according one of claims 1 to 3, characterized in that the two short sides (6) of the rectangle are upwardly slightly convergent.

5. Anklet according to one of claims 1 to 4, characterized in that the fabric part (1) is closed on itself in an inseparable manner, for example by sewing (7).

6. Anklet according to one of claims 1 to 4, characterized in that the fabric part (1) is closed on itself in a separable manner, for example by a self-gripping type fastener.

7. Anklet according to one of claims 1 to 6, characterized in that the reinforcement part (2) is substantially triangular, the corner portions (8, 9) of the triangle being intended to cover the Achilles tendon upper portion and the two malleoluses, respectively.

8. Anklet according to one of claims 1 to 7, characterized in that the reinforcement part (2) is made of thermocompressed foam.

9. Anklet according to one of claims 1 to 8, characterized in that the reinforcement part (2) comprises depressed lines (11, 12) giving flexibility thereto.

10. Anklet according to one of claims 1 to 9, characterized in that the reinforcement part (2) comprises a central vertical channel in the zone of cooperation with the Achilles tendon.

11. Anklet according to one of claims 1 to 10, characterized in that the reinforcement part (2) is secured on the outer face of the fabric part (1), for example by sewing.

12. Anklet according to one of claims 1 to 11, characterized in that the fabric part (1) is adapted to receive a tibia-protector (14), in a separable or fixed manner, for example by sewing (15) on the upper front edge of the fabric part (1).

## Patentansprüche

1. Knöchelsocke, insbesondere für den Sport, welche einerseits ein elastisches Gewebestück (1), das flach hergestellt und zur Bildung einer Röhre in sich selbst geschlossen ist, und andererseits ein weiches Stück (2) umfaßt, welch letzteres an dem Gewebestück (1) befestigt und derart angeordnet ist, daß es zumindest die Achillessehne bedeckt, dadurch gekennzeichnet, daß das Gewebestück (1) eine annähernd rechteckige Form besitzt und an einer Längsseite eine mittige Ausbuchtung (3) zur Bedeckung des oberen Abschnittes der Achillessehne sowie an der entgegengesetzten Längsseite zwei voneinander beabstandete und annähernd symmetrische Ausbuchtungen (4) zur Bedeckung der Knöchel aufweist, und daß das weiche Stück (2) ein Verstärkungs- und Schutzelement ist, welches ebenfalls die Knöchel bedeckt.

2. Knöchelsocke nach Anspruch 1, dadurch gekennzeichnet, daß das Gewebestück (1) mindestens in Querrichtung der Röhre elastisch ist.

3. Knöchelsocke nach Anspruch 1 oder 2, dadurch gekennzeichnet, daß das Gewebestück (1) durch ein Strickgewebe gebildet ist, das glatt gewirkt und geschnitten ist.

4. Knöchelsocke nach einem der Ansprüche 1 bis 3, dadurch gekennzeichnet, daß die zwei kurzen Seiten (6) des Rechtecks nach oben zu leicht konvergent sind.

5. Knöchelsocke nach einem der Ansprüche 1 bis 4, dadurch gekennzeichnet, daß das Gewebestück (1) in sich selbst unlösbar geschlossen ist, beispielsweise durch Vernähen.

6. Knöchelsocke nach einem der Ansprüche 1 bis 4, dadurch gekennzeichnet, daß das Gewebestück (1) in sich selbst lösbar geschlossen ist, beispielsweise durch einen selbsthaftenden Verschluß.

7. Knöchelsocke nach einem der Ansprüche 1 bis 6, dadurch gekennzeichnet, daß das Verstärkungsstück (2) annähernd dreieckig ist, wobei die Eckabschnitte (8, 9) des Dreiecks dazu bestimmt sind, jeweils den oberen Teil der Achillessehne bzw. die beiden Knöchel zu bedecken.

8. Knöchelsocke nach einem der Ansprüche 1 bis 7, dadurch gekennzeichnet, daß das Verstärkungsstück (2) aus warmverdichtetem Schaum besteht.

9. Knöchelsocke nach einem der Ansprüche 1 bis 8, dadurch gekennzeichnet, daß das Verstärkungsstück (2) Hohllinien (11, 12) aufweist, welche ihm seine Weichheit verleihen.

10. Knöchelsocke nach einem der Ansprüche 1 bis 9, dadurch gekennzeichnet, daß das Verstärkungsstück (2) in der Zone des Zusammenwirkens mit der Achillessehne einen mittigen, senkrechten Kanal aufweist.

11. Knöchelsocke nach einem der Ansprüche 1 bis 10, dadurch gekennzeichnet, daß das Verstärkungsstück (2) auf der Außenoberfläche des Gewebestücks (1), beispielsweise durch Nähen, befestigt ist.

12. Knöckelsocke nach einem der Ansprüche 1 bis 11, dadurch gekennzeichnet, daß das Gewebestück (1) zur Aufnahme eines Schienbeinschoners, abnehmbar oder fest, beispielsweise durch eine Naht (15) am oberen vorderen Rand des Gewebestücks (1), eingerichtet ist.
